# EUROPÄISCHE PATENTANMELDUNG

(11) **EP 1 762 263 A1**
(43) Veröffentlichungstag der Anmeldung: **14.03.2007**
(21) Anmeldenummer: 05019573.4
(22) Anmeldetag: 08.09.2005
(51) Int. Cl.: A61M 5/36, G01F 1/74, A61M 5/142

(54) **Blasendetektor, Okklusionsdetektor oder Leckdetektor in einer oder für eine Vorrichtung zur Verabreichung eines flüssigen Produkts**

(71) Anmelder: Hoffman-La Roche AG, 4070 Basel (CH); Roche Diagnostics GmbH, 68305 Mannheim (DE)
(72) Erfinder: Remde, Axel, 3432 Lützelflüh (CH)
(74) Vertreter: Schwabe - Sandmair - Marx

(57) **Zusammenfassung**

Elektrodenanordnung mit einer ersten Messelektrode (3) und einer zweiten Messelektrode (4), die in einem Strömungskanal (1) in einem Abstand voneinander angeordnet sind und mit einem flüssigen Produkt (P) bei Durchströmung des Strömungskanals (1) einen elektrischen Kondensator (C) oder Ohm'schen Widerstand (R) bilden, wobei die Elektrodenanordnung (3, 4) für die Verwendung als Sensor für die Detektion einer von dem Produkt (P) mitgeführten Gasblase (B), einer Okklusion oder eines Lecks in einer Vorrichtung für die Verabreichung des Produkts (P) vorgesehen ist.

## Beschreibung

Die Erfindung betrifft eine Vorrichtung zur Verabreichung eines flüssigen Produkts mit einer Elektrodenanordnung für die Detektion von in dem Produkt mitgeführten Gasblasen oder einer Okklusion oder eines Lecks in einem das Produkt führenden Fluidsystem der Vorrichtung. Die Erfindung betrifft ferner die Elektrodenanordnung als solche, die für eine Verwendung als Sensor für die Detektion einer Gasblase, einer Okklusion oder eines Lecks im Fluidsystem solch einer Vorrichtung vorgesehen ist. Die Erfindung kann insbesondere in der Selbstverabreichung eines Medikaments, beispielsweise Insulin, eingesetzt werden.

In der Verabreichung von Medikamenten ist die exakte Dosierung wesentlich. Dabei stellen nicht nur unbemerkte Okklusionen oder Lecks eine Gefahr dar, sondern auch im flüssigen Medikament gelöste Gase, insbesondere Luft, die unter den üblichen Betriebsbedingungen von Verabreichungsvorrichtungen Gasblasen bilden und das tatsächlich geförderte Volumen des wirksamen Produkts entsprechend dem Gasanteil verringern.

Es ist eine Aufgabe der Erfindung, bei der Verabreichung eines flüssigen Produkts von dem Produkt mitgeführtes Gas oder das Auftreten einer Okklusion oder eines Lecks feinfühlig zu detektieren.

Eine Vorrichtung zur Verabreichung eines flüssigen Produkts, wie die Erfindung sie betrifft, umfasst eine Fördereinrichtung, einen Strömungskanal, durch den das Produkt mittels der Fördereinrichtung förderbar ist, und eine Messeinrichtung. Die Messeinrichtung umfasst eine erste Messelektrode und eine zweite Messelektrode, einen Spannungserzeuger zur Erzeugung einer elektrischen Potenzialdifferenz zwischen den Messelektroden und eine Verarbeitungseinrichtung, die zur Bildung eines Messsignals mit den Messelektroden verbunden ist. Die Messelektroden sind in einem Abstand voneinander angeordnet und werden bei Durchströmung des Strömungskanals über das Produkt elektrisch miteinander gekoppelt. Einander zugewandte Flächen der Elektroden, zwischen denen das Produkt hindurchströmt, haben voneinander einen Abstand vorzugsweise aus dem Bereich von 0.5 bis 2mm. Die Messelektroden und das den Abstand überbrückende Fluid, im Betrieb das Produkt oder auch nur eine die Messelektroden passierende Gasblase, bilden in einer Messschaltung der Verarbeitungseinrichtung einen Ohm'schen Widerstand oder einen elektrischen Kondensator mit dem Fluid als Dielektrikum, wobei die Verarbeitungseinrichtung entweder nur den Wert des Ohm'schen Widerstands oder nur den Wert der Kapazität oder beides in Kombination ermittelt. Die Erfindung nutzt zur Messung die elektrischen Eigenschaften des Produkts.

Das Wort "oder" umfasst im Sinne der Erfindung die Bedeutung des Wortes "und", wird also der formalen Logik entsprechend verwendet, soweit sich aus dem jeweiligen Zusammenhang nichts anderes ergibt.

Wegen der unterschiedlichen elektrischen Leitfähigkeiten und dem Unterschied in der jeweiligen relativen Dielektrizitätszahl der einem Menschen oder einem Tier zu therapeutischen, diagnostischen oder pharmazeutischen Zwecken verabreichbaren Produktflüssigkeiten, die meist auf Wasser basiert sind, und der in den Produktflüssigkeiten mitgeführten Gase, vor allem Luft, ermöglicht die Erfindung insbesondere die Detektion von Gasblasen und somit die Schaffung eines Blasendetektors.

Falls die Messelektroden so angeordnet oder als solche so gebildet sind, dass sich ihr Abstand zueinander in Abhängigkeit von dem statischen Druck im Strömungskanal ändert, können die Messelektroden anstatt eines Blasendetektors oder zusätzlich einen Sensor für die Detektion einer Okklusion oder eines Lecks bilden.

Die Erfindung hat ferner eine Elektrodenanordnung der beschriebenen Art als solche zum Gegenstand, soweit die Elektrodenanordnung als Sensor für die Detektion von den Strömungskanal durchströmenden Blasen oder einer Okklusion oder eines Lecks im Strömungskanal stromauf oder stromab von der Elektrodenanordnung verwendet wird oder für eine derartige Verwendung vorgesehen ist. Die Elektrodenanordnung als solche oder als Bestandteil der Verabreichungsvorrichtung kann insbesondere in einer den Strömungskanal bildenden flexiblen Kanüle angeordnet sein. In bevorzugter Ausführung ist die Elektrodenanordnung allein oder gemeinsam mit dem Spannungserzeuger oder gemeinsam mit der Verarbeitungseinrichtung oder einem Teil der Verarbeitungseinrichtung in einem eigenen Gehäuse aufgenommen. Das Gehäuse mit der darin aufgenommen Elektrodenanordnung oder der gesamten Messeinrichtung oder einem Teil der Messeinrichtung einschließlich Elektrodenanordnung kann insbesondere mit einer Verbindungseinrichtung für eine feste Verbindung mit einem Gehäuse der Verabreichungsvorrichtung versehen sein. Die Verbindung zwischen dem Gehäuse der Elektrodenanordnung und dem Gehäuse der Verabreichungsvorrichtung kann rein formschlüssig oder rein kraftschlüssig oder form- und kraftschlüssig und im Grunde sogar stoffschlüssig sein. Die Verbindung kann insbesondere eine Schraubverbindung oder eine Rastverbindung sein. In einer alternativen, ebenfalls bevorzugten Ausführung, bildet das die Elektrodenanordnung aufnehmende Gehäuse einen Katheterkopf, der auf der Haut nahe dem Ort der Verabreichung platzierbar und vorzugsweise befestigbar ist. Die Verabreichung erfolgt bevorzugt subkutan, kann aber auch intravenös oder auch in tiefergelegenes Gewebe vorgenommen werden.

Für die Detektion einer Blase kann die eine der Messelektroden stromabwärts von der anderen angeordnet sein, gegebenenfalls nur in Strömungsrichtung des Produkts voneinander beabstandet. In solchen Ausführungen sollte der Abstand nicht größer sein, als die in Strömungsrichtung gemessene Länge der zu detektierenden Gasblasen.

In bevorzugten Ausführungen überlappen die Messelektroden einander in Strömungsrichtung zumindest teilweise und sind einander quer zu der Strömungsrichtung gegenüberliegend angeordnet.

Vorzugsweise ist wenigstens eine der Messelektroden in einer Seitenberandung des Strömungskanals angeordnet. Noch bevorzugter sind die erste und die zweite Messelektrode in der Seitenberandung angeordnet. Die in der Seitenberandung angeordnete Elektrode oder Elektroden kann oder können über die vorzugsweise glatte Seitenberandung in die Strömung vorragen. Eine oder beide Messelektroden können auch hinter der Mantelinnenfläche der Seitenberandung zurückstehen. Besonders bevorzugt bilden die Elektroden aber je eine Teilfläche der in diesem Fall auch über die betreffende Elektrode glatten Seitenberandung, d. h. sie sind in die Seitenberandung bündig eingebettet.

Für die Bestimmung der Volumina von Blasen ist es vorteilhaft, wenn die Messelektroden oder auch nur eine der Messelektroden in Strömungsrichtung eine geringe Länge aufweist oder aufweisen, d. h. möglichst schmal ist oder sind. Andererseits ist es für die praktischen Belange vorteilhaft, wenn die Messelektroden eine gewisse flächenhafte Ausdehnung aufweisen. Die Messelektroden weisen daher in Strömungsrichtung in vorteilhaften Ausführungen eine Länge von wenigsten 0.5mm und vorzugsweise höchstens 5mm auf. Handelt es sich bei dem Produkt beispielsweise um Insulin, sollte die in Strömungsrichtung gemessene Länge der Messelektroden je wenigstens so groß sein, dass eine Gasblase, die den Querschnitt des Strömungskanals zwischen den Messelektroden gerade vollständig ausfüllt, ein Volumen aufweist, das höchstens dem Volumen der Insulinlösung oder -suspension mit höchstens einer Insulineinheit, bevorzugt höchstens einer halben Insulineinheit entspricht. Andererseits sollte das Volumen nicht kleiner sein als das Volumen der Insulinlösung oder -suspension, die einem Zehntel einer Insulineinheit entspricht. Für ein Insulin üblicher Konzentration, beispielsweise U100, bedeutet dies, dass das Volumen zwischen den Messelektroden wenigstens 0.1 und höchstens 1mm³ sein sollte. Ist der Strömungskanal beispielsweise kreisrund mit einem Durchmesser von 0.5mm, sollten die Messelektroden eine Länge von wenigstens 0.5mm und höchstens 5mm haben. Für die Praxis vorteilhaft ist eine Länge von 2mm +/- 1 Millimeter, was im Falle der beispielhaft angenommenen Konzentration U100 bedeutet, dass die den Strömungsquerschnitt zwischen den Messelektroden gerade vollständig ausfiillende Gasblase ein Volumen von etwa 0.4mm³ +/-0.2mm³ hat.

Um ein möglichst großes Messsignal zu erhalten, ist es vorteilhaft, wenn die Messelektroden quer zu der Strömungsrichtung möglichst breit sind und in Strömungsrichtung eine große, bevorzugt vollständige Überlappung aufweisen. So können die Messelektroden sich vorteilhafterweise je über einen im Querschnitt des Strömungskanals gemessenen Umfangswinkel von nahezu 180° erstrecken. Es kann auch eine der Elektroden zentral im Strömungskanal angeordnet sein und die andere Elektrode diese innere Elektrode zum Teil oder vorteilhafterweise über den gesamten Umfang umgeben.

In ersten Ausführungen besteht zwischen den Messelektroden und dem Produkt im Falle der Durchströmung eine galvanische Kopplung d. h. die Messelektroden sind galvanisch koppelbar. In zweiten Ausführungen sind die Messelektroden über das Produkt nicht galvanisch gekoppelt. Sie sind galvanisch getrennt, indem wenigstens eine der Messelektroden gegen das Produkt elektrisch isoliert ist, so dass die Messelektroden über das Produkt nur kapazitiv gekoppelt sind. In der zweiten Ausführung können die wenigstens eine isolierte Messelektrode oder vorzugsweise die beiden isolierten Messelektroden nichtleitend beschichtet sein, beispielsweise durch Bedampfung oder Bedrucken oder Laminieren mit einem Isolationsmaterial. Ein Leitermaterial, beispielsweise ein metallisches Material, kann auch in einem Isolationsmaterial, beispielsweise einem Kunststoff oder einem keramischen Material, eingebettet sein. Metalloxide sind bevorzugte Isolationsmaterialien. Die Seitenberandung kann ebenfalls die Isolation bilden, indem sie eine oder beide Messelektroden zum Strömungskanal hin bedeckt. Die elektrische Isolierung sollte möglichst dünn oder ihre relative Dielektrizitätszahl möglichst groß sein, um die elektrische Kapazität der Elektrodenanordnung so wenig wie möglich zu verringern.

Die Messelektroden können als metallische Festkörper mit oder ohne Isolierung oder durch partielle Metallisierung eines isolierenden Substrats gebildet sein, wobei im letzteren Fall als Substrat insbesondere elastische Membranen oder Folien geeignet sind. Das Substrat kann entweder auf der vom Produkt benetzten Innenseite mit der Folge einer galvanischen Kopplung oder auf der trockenen Außenseite mit der Folge rein kapazitiver Kopplung metallisiert sein.

Bildet die Elektrodenanordnung einen Okklusionsdetektor oder Leckdetektor, entweder in alleiniger Funktion oder in Kombination mit der Funktion als Blasendetektor, werden die Messelektroden einander quer zu der Strömungsrichtung gegenüberliegend so angeordnet oder gestaltet, dass der zwischen ihnen bestehende Abstand sich bei einer mit einer Okklusion oder einem Leck verbundenen Druckänderung ebenfalls ändert. In einer ersten Variante ist die erste Messelektrode in einer elastischen Seitenberandung des Strömungskanals befestigt. Vorzugsweise ist auch die zweite Messelektrode in einer elastischen Seitenberandung des Strömungskanals befestigt. Zweckmäßigerweise begrenzt die elastische Seitenberandung den Strömungskanal im Bereich der Elektrodenanordnung über seinen gesamten Umfang. Der Abstand zwischen den Elektroden vergrößert sich im Falle einer Okklusion entsprechend der elastischen Aufweitung des Strömungskanals. Bei Auftreten eines Lecks verringert sich der Abstand entsprechend dem Druckabfall. Anstatt oder zusätzlich zu der Befestigung in oder an einer elastischen Seitenberandung kann die erste Messelektrode auch als solche elastisch nachgiebig gestaltet sein, beispielsweise als elastische Membran, die sich entsprechend den Druckverhältnissen im Strömungskanal mehr oder weniger wölbt, so dass sich zumindest ein mittlerer Abstand zwischen den Messelektroden entsprechend den Druckverhältnissen ändert. Zusätzlich kann auch die zweite Messelektrode als solche elastisch, beispielsweise als elastische Membran, gebildet sein.

In einer alternativen Ausführung ist für die Detektion einer Okklusion oder eines Lecks eine weitere Elektrodenanordnung vorgesehen, die wie die beschriebene Elektrodenanordnung gebildet sein kann. Die erste und die zweite Messelektrode dienen in solchen Ausführungen nur oder primär der Detektion von Gasblasen, während die weitere Elektrodenanordnung der Detektion einer Okklusion oder eines Lecks dient. Angesichts dieser Aufgabenteilung ist es allerdings vorteilhaft, wenn die Messelektroden der weiteren Elektrodenanordnung einander in Strömungsrichtung des Produkts über eine größere Länge überlappen. Eine der Messelektroden der weiteren Elektrodenanordnung kann gleichzeitig auch die erste oder die zweite Messelektrode bilden, d. h. die beiden Elektrodenanordnungen können vier separate Messelektroden oder insgesamt nur drei Messelektroden umfassen, von denen eine Messelektrode beider Elektrodenanordnungen ist.

Die Fördereinrichtung der Verabreichungsvorrichtung umfasst ein für die Förderung des Produkts auf das Produkt wirkendes Förderglied, vorzugsweise einen Kolben, der in einem Produktreservoir in eine Vortriebsrichtung bewegbar angeordnet ist, und einen Antrieb für das Förderglied. Der Antrieb kann insbesondere als elektrischer Drehmotor gebildet sein. Für die Übertragung der Antriebsbewegung des Antriebs sind der Antrieb und das Förderglied vorzugsweise über ein Getriebe, das insbesondere als Spindeltrieb gebildet oder einen Spindeltrieb umfassen kann, miteinander gekoppelt.

Die Verabreichungsvorrichtung umfasst in bevorzugter Ausführung eine Regelungseinrichtung für die Regelung der Förderrate oder nur der absoluten Fördermenge pro Verabreichungsvorgang. Die Regelungseinrichtung umfasst einen Regler und als Stellglied den Antrieb der Fördereinrichtung mit dem nachgeschalteten Förderglied. Ferner umfasst sie die Messeinrichtung. Falls die Messeinrichtung wie bevorzugt einen Blasendetektor bildet, ist ein Ausgang der Verarbeitungseinrichtung mit einem Eingang des Reglers verbunden, um dem Regler das Messergebnis des Blasendetektors als eine Eingangsgröße aufzugeben. Die Regelungseinrichtung regelt den Antrieb in Abhängigkeit von der von der Verarbeitungseinheit erhaltenen Eingangsgröße. Diese Eingangsgröße kann die Regelgröße der Regelungseinrichtung bilden oder auch eine zusätzliche Eingangsgröße. Sollte der Antrieb von Hause aus nicht geregelt, sondern nur gesteuert werden, wird durch die Aufschaltung der Ausgangsgröße der Verarbeitungseinrichtung als dann einzige zurückgeführte Größe eine Regelungseinrichtung erst erhalten. Sollte der Antrieb von Hause aus bereits geregelt werden, beispielsweise durch Rückführung einer Drehwinkelposition eines Drehmotors als die Regelgröße, so bildet die Ausgangsgröße der Verarbeitungseinheit eine zusätzliche Eingangsgröße für den Regler, beispielsweise in Form einer Störgröße.

Die Messeinrichtung kann die passierenden Gasblasen ganz einfach nur zählen und jedes Ereignis, d. h. jede Zählung einzeln als die Ausgangsgröße bilden oder mit einem Addierwerk ausgestattet sein, um die Anzahl der Gasblasen kontinuierlich zu zählen und jeweils nach einem bestimmten Zeitintervall in der Summe oder pro Verabreichungsvorgang zu zählen und in der Summe als die Ausgangsgröße auszugeben.

In einer Weiterentwicklung berechnet die Verarbeitungseinrichtung das Volumen der Gasblasen. Hierfür stellt sie den Zeitpunkt fest, zu dem der Ohm'sche Widerstand oder die Kapazität sich in der für die Passage einer Gasblase charakteristischen Weise ändert oder ändern und stellt ferner den Zeitpunkt fest, zu dem der Ohm'sche Widerstand oder die Kapazität wieder auf den Wert abfällt oder abfallen, der für das Produkt selbst charakteristisch ist. Im normalen Betriebszustand, d. h. im okklusionsfreien und leckfreien Zustand, kann der Querschnitt des Strömungskanals nach Form und Fläche als konstant und somit als vorgegeben angesehen werden. Ferner kann im normalen Betriebszustand auch die Strömungsgeschwindigkeit als bekannt, da durch die vorbestimmte Fördertätigkeit der Fördereinrichtung vorgegeben, angesehen werden. Aus der Differenz der festgestellten Zeitpunkte, nämlich Eintritt einer Gasblase und Austritt der Gasblase in und aus dem Bereich der Elektrodenanordnung, dem bekannten Querschnitt und der bekannten Strömungsgeschwindigkeit kann das Volumen der betreffenden Gasblase auf einfache Weise ermittelt werden. Gegebenenfalls kann ein Sensor für die Ermittlung der Strömungsgeschwindigkeit separat vorgesehen und mit der Verarbeitungseinrichtung verbunden sein.

In einer Variante der Regelungseinrichtung bildet die Verarbeitungseinrichtung eine dem Volumen einer einzelnen Gasblase entsprechende Ausgangsgröße und gibt diese dem Regler als Eingangsgröße auf. In einer anderen Variante ist die Verarbeitungseinrichtung mit einem Addierwerk ausgestattet, das die Volumina der detektierten Gasblasen summiert, und die Verarbeitungseinrichtung bildet ihre Ausgangsgröße für den Regler als Summensignal.

Falls das Ausgangssignal der Verarbeitungseinrichtung jeweils ein Zählsignal oder ein Volumensignal für jede einzelne Gasblase ist, passt der Regler seine Stellgröße für den Antrieb der Fördereinrichtung augenblicklich oder zeitverzögert an, d. h. es wird die Förderrate augenblicklich oder zeitverzögert entsprechend dem aus den Gasblasen reduzierenden Defizit eingestellt. Die Ausgangsgröße der Verarbeitungseinrichtung kann auch ein Summensignal sein, entweder die Gesamtzahl der pro vorgegebener Zeiteinheit oder pro Verabreichungsvorgang gezählten Blasen oder des ermittelten Gesamtvolumens der Blasen. Anstatt oder zusätzlich zu einer Erhöhung der Förderrate kann es auch vorteilhaft sein, wenn der Regler die Stellgröße so bildet, dass die Fördertätigkeit der Fördereinrichtung das Defizit kompensierend verlängert wird. Diese Art der Regelung kann insbesondere dann angewendet werden, wenn die Verabreichung unterscheidbare Verabreichungsvorgänge umfasst oder nur in solchen erfolgt. In der Diabetestherapie mit ständig am Körper tragbaren Infusionsgeräten beispielsweise wird das Produkt mit einer Basalrate und zu besonderen Ereignissen in Form von Sonderboli verabreicht. In solchen Anwendungen wird die Basalrate beispielsweise entsprechend dem ermittelten Defizit angehoben, während ein bei Verabreichung eines Sonderbolus festgestelltes Defizit vorzugsweise an den Sonderbolus angehängt wird, vorzugsweise durch Förderung bei gleicher Förderrate über ein berechnetes zusätzliches Zeitintervall.

Zur Kompensation des durch eine Gasblase entstandenen Defizits wird dabei bevorzugt wie folgt vorgegangen: Erkennt der Detektor eine Luftblase, wird zunächst die Förderung weiter durchgeführt, bis das Ende des Detektors das stromaufwärtige Ende der Blase detektiert hat. Daraufhin ermittelt die Verarbeitungseinrichtung das Volumen der Blase. Anschließend wird zur Kompensation des durch die Blase entstandenen Defizits die basale Förderrate für einen gewissen, von der Bearbeitungseinrichtung ermittelten Zeitraum angehoben. Das Defizit wird nicht schlagartig kompensiert, sondern über einen gewissen Zeitraum. Auf diese Weise wird vermieden, dass es im Anschluss an die Unterförderung zu einer starken Überförderung mit gegebenenfalls negativen Auswirkungen auf den Patienten kommen kann. Die für die Kompensation genutzte Zeitspanne kann entweder fest vorgegeben sein, zum Beispiel eine halbe Stunde, oder in Abhängigkeit von der aktuellen Förderrate festgelegt werden. So kann es sinnvoll sein, die Korrektur so durchzuführen, dass die nominelle Förderrate, das heißt die Förderrate ohne Berücksichtigung der Korrektur, höchstens verdoppelt wird. Aber auch die Verabreichung eines im Volumen der Blase entsprechenden, zeitnah mit der Blase verabreichten Sonderbolus kann vorteilhaft sein.

Für die Kompensation des Defizits wird vorzugsweise auch die Anordnung des Detektors im Strömungskanal berücksichtigt, um eine Zeitverzögerung zu berechnen, nach der die Kompensation durchgeführt wird. Wird der Detektor nahe am Ort der Verabreichung, beispielsweise in oder nahe bei einer Einstechkanüle angeordnet, so fallen der Zeitpunkt, zu dem die Blase detektiert wird, und der Zeitpunkt, zu dem sie in den Körper injiziert wird, praktisch zusammen. Entsprechend sollte die Kompensation sofort im Anschluss an die Detektion erfolgen. Ist der Detektor dagegen nahe bei der Fördereinrichtung angeordnet und sind Fördereinrichtung und Kanüle durch eine Infusionsleitung miteinander verbunden, entsteht zwischen Detektion und Infusion der Blase ein Zeitversatz, da vor der Blase noch die zum Zeitpunkt der Detektion in der Infusionsleitung enthaltene Produktmenge infundiert wird. Entsprechend darf die Kompensation erst zu dem Zeitpunkt erfolgen, zu dem die Blase die Kanüle passiert.

So kommt es in der Selbstverabreichung von Medikamenten, beispielsweise Insulin, ohne weiteres vor, dass ein bestimmtes Produktvolumen vom Auslass eines Produktreservoirs bis zum Ende des Strömungskanals, das heißt bis zum Ort der Verabreichung, ein Zeitintervall von einer oder auch mehreren Stunden benötigt. Sind die Messelektroden nahe bei dem Reservoir angeordnet, so dass eine die Messanordnung passierende Gasblase bis zum Ort der Verabreichung ein entsprechend langes Zeitintervall benötigt, so wird das Mindervolumen an Medikament idealerweise erst dann nachgefördert, wenn die Gasblase den Ort der Verabreichung erreicht, im Idealfall, wenn die Gasblase gerade den Strömungskanal an dessen Mündung verlassen hat. Grundsätzlich gilt dies nicht nur bei den beispielhaft angegebenen langen Zeitintervallen, sondern auch für kürzere Zeitintervalle. In vorteilhaften Weiterbildungen der Erfindung ermittelt die Verarbeitungseinrichtung das Zeitintervall, beispielsweise in Abhängigkeit von der Strömungsgeschwindigkeit und dem der Verarbeitungseinrichtung bekannten Ort der Messanordnung auf dem Strömungsweg des Produkts. In einer vereinfachten Ausführung kann das Zeitintervall auch in einem Datenspeicher fest vorgegeben sein. Die Ermittlung des Zeitintervalls oder alternativ nur die Vorgabe als fester Speicherwert wird vorliegend zwar der Verarbeitungseinrichtung zugeordnet, insoweit kann die Verarbeitungseinrichtung jedoch Bestandteil des Reglers für den Antrieb der Fördereinrichtung sein.

An die Messelektroden wird bevorzugt eine Wechselspannung angelegt, wodurch vorteilhafterweise Elektrolysereaktionen im Produkt verhindert oder gegenüber einer Beaufschlagung mit Gleichspannung zumindest reduziert werden können. Die Frequenz der Wechselspannung sollte wenigstens 10 Hz und kann bis zu 100 kHz betragen, falls nur der Ohm'sche Widerstand gemessen wird. Im Falle der Messung nur der Kapazität oder sowohl des Ohm'schen Widerstands als auch der Kapazität ist eine Frequenz aus dem Bereich von 100 Hz bis 200 kHz vorteilhaft.

Der Spannungserzeuger umfasst eine Energiequelle, vorzugsweise mit einem Energiespeicher, beispielsweise eine elektrische Batterie oder ein Brennstoffreservoir einer Brennstoffzelle. Im Falle der bevorzugten Beaufschlagung mit einer Wechselspannung ist die Spannungsquelle über einen Oszillator oder Generator mit der Elektrodenanordnung verbunden.

Bei der Energiequelle kann es sich um die Energiequelle für den genannten Antrieb der Fördereinrichtung handeln. Alternativ kann die Messeinrichtung auch von einer eigenen, separaten Energiequelle, die vorzugsweise als elektrische Miniaturbatterie, gebildet ist, mit Energie versorgt werden. Eine unabhängige Energieversorgung der Messeinrichtung oder nur der Elektrodenanordnung kann insbesondere dann von Vorteil sein, wenn die Messeinrichtung oder nur die Elektrodenanordnung mit dem Spannungserzeuger Bestandteil eines unmittelbar auf der Haut platzierten Katheterkopfs der Verabreichungsvorrichtung ist, da derartige Katheterköpfe üblicherweise lediglich über eine flexible Kanüle mit dem Rest der Vorrichtung, insbesondere dem Produktreservoir der Fördereinrichtung, verbunden sind. Falls die Energiequelle für die Fördereinrichtung gleichzeitig auch die Energiequelle für die Elektrodenanordnung bildet, kann auf besonders einfache Weise sichergestellt werden, dass die Elektrodenanordnung nur während der Fördertätigkeit der Fördereinrichtung mit Energie beaufschlagt wird, indem die Energie für die Elektrodenanordnung im Energieversorgungskreis erst hinter oder gleichzeitig mit der Versorgung der Fördereinrichtung abgezweigt wird: Falls die Fördereinrichtung mit Gleichstrom betrieben wird, kann eine beispielsweise rotierende Komponente der Fördereinrichtung gleichzeitig einen Generator und somit einen Wechselspannungserzeuger für die Elektrodenanordnung bilden. Falls die Fördereinrichtung mit Wechselspannung betrieben wird, kann die Elektrodenanordnung mit der gleichen Frequenz wie die Fördereinrichtung und somit insbesondere über eine gegebenenfalls bereits für die Fördereinrichtung vorgesehene Oszillatorschaltung beaufschlagt werden.

Um den Energieverbrauch der Elektrodenanordnung und auch die Erwärmung des Produkts gering zu halten, wird die Elektrodenanordnung vorteilhafterweise nicht ständig betrieben, sondern nur dann, wenn Produkt gefordert wird. Gegebenenfalls wird die Elektrodenanordnung auch während der Förderung des Produkts jeweils nur kurzzeitig in einem vorgegebenen Zeittakt, d. h. periodisch, beispielsweise pro Minute ein Mal über wenige Sekunden oder nur Millisekunden betrieben.

Jedenfalls sollte der Detektor immer dann betrieben werden, wenn die Fördereinrichtung einen Produktbolus fördert. Im Modus einer Basalratenförderung kann der Detektor mit der Fördereinrichtung vorteilhafterweise synchronisiert betrieben werden, insbesondere in denjenigen Fällen, in denen die Fördereinrichtung auch die Basalrate getaktet fördert, nämlich das jeweils gleiche Produktvolumen in periodisch vorgegebenen Zeitabständen. Wird das bestimmte Produktvolumen im Rahmen einer Basalratenförderung beispielsweise alle drei Minuten gefördert, kann auch der Detektor synchron, das heißt gleichzeitig mit der Fördereinrichtung betrieben werden. Jedenfalls sollte der Detektor wenigstens während der aktiven Fördertätigkeit der Fördereinrichtung betrieben werden. Zusätzliche Aktivierungen des Detektors sind insbesondere dann von Vorteil, wenn zwischen Förderzeiten der Fördereinrichtungen auch Stillstandszeiten längerer Dauer vorliegen, beispielsweise bei einer Basalratenförderung im Dreiminutentakt. In solchen Fällen ist es vorteilhaft, wenn der Detektor nicht nur während der aktiven Förderzeit der Fördereinrichtung, sondern auch dazwischen, beispielsweise pro Minute wenigstens einmal, eingeschaltet ist. Solche zusätzlichen Aktivierungen des Detektors sind im Hinblick darauf sinnvoll, dass durch hydrostatische Druckunterschiede und von außen einwirkende Trägheitskräfte Bewegungen des Fluids auch ohne Fördertätigkeit der Fördereinrichtung möglich sind. Im Falle eines diskontinuierlich betriebenen Detektors besteht die Gefahr, dass der Blasendetektor in Zeiträumen der Deaktivierung von einer Gasblase passiert wird, ohne dass die Blase detektiert würde. Im Hinblick hierauf bietet ein kontinuierlich betriebener Detektor die größte Sicherheit.

Vom Standpunkt der Verabreichung ausgesehen ist es vorteilhaft, wenn die Messanordnung nahe bei dem Ort der Verabreichung angeordnet ist. So kann die Messanordnung beispielsweise Bestandteil eines Kathederkopfs sein, der am Ort der Verabreichung auf der Haut platziert ist und als Träger einer das Gewebe infundierenden Einführeinrichtung dient. Die Einführrichtung bildet das Ende des Strömungskanals. Im Falle der Anordnung der Messanordnung in solch einem Kathederkopf kommuniziert die Messanordnung entweder drahtlos oder vorzugsweise leitungsgebunden mit der Pumpe. Die Verarbeitungseinrichtung ist vorzugsweise Bestandteil der Pumpe. Die Leitung oder mehreren Leitungen können insbesondere in der Wandung des Strömungskanals eingebetet sein. Falls die Verarbeitungseinrichtung oder ein Teil davon ebenfalls Bestandteil des Kathederkopfs ist, findet die Kommunikation zwischen Verarbeitungseinrichtung und dem vorzugsweise vorhandenen Regler einer Fördereinrichtung statt. In alternativen Ausführungen, die ebenfalls bevorzugt sind, ist die Messanordnung Bestandteil der Pumpe, das heißt sie ist innerhalb des Gehäuses der Pumpe oder in einem Anbauteil, das unmittelbar an dem Gehäuse befestigt ist, untergebracht. Ein derartiges Anbauteil ist vorzugsweise lösbar mit dem Gehäuse der Pumpe verbunden. Die Messanordnung ist vorzugsweise als Wegwerfteil konzipiert, das nach Gebrauch gegen ein neues ausgetauscht wird. So kann die Messanordnung beispielsweise jeweils zusammen mit einer Medikamentenampulle ausgetauscht werden.

Vorteilhaft ist es ferner, wenn die Vorrichtung eine Alarmeinrichtung aufweist. Insbesondere bei Auftreten von besonders großen Gasblasen oder einer dichten Abfolge vieler Gasblasen kann es vorteilhaft sein, wenn anstatt oder zusätzlich zu einer Kompensation mittels der Fördereinrichtung eine Alarmierung des Benutzer erfolgt. Die Alarmierung kann akustisch, optisch oder taktil erfolgen, einschließlich jeder Kombination derartiger Alarmsignale. Die Alarmierung kann nach unterschiedlichen Kriterien vorgenommen werden. Kriterien für die Auslösung eines Alarms können insbesondere sein: die Infusion einer besonders großen Einzelblase, im Falle von Insulin beispielsweise fünf Insulineinheiten (5 IU), die Infusion einer großen Luftmenge innerhalb eines bestimmten Zeitintervalls zum Beispiel innerhalb einer Stunde, oder die Infusion einer Gasmenge, deren Kompensation eine lange Zeit in Anspruch nähme, beispielsweise mehr als eine oder alternativ zwei Stunden, wobei die Kompensation in Abhängigkeit von der programmierten oder alternativ hardwareseitig vorgegebenen Förderrate der Fördereinrichtung abhängt. Bevorzugt wird es, wenn die Alarmierung im Falle jedes der drei genannten Ereignisse vorgenommen wird. Die Alarmeinrichtung ist signaltechnisch entsprechend mit der Verarbeitungseinrichtung verbunden.

Vorteilhafte Merkmale der Erfindung werden auch in den Unteransprüchen und deren Kombinationen beschrieben.

Ausführungsbeispiele der Erfindung werden nachfolgend anhand von Figuren erläutert. An den Ausführungsbeispielen offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: einen von einer Produktflüssigkeit durchströmten Strömungskanal mit einer Elektrodenanordnung in einem Längsschnitt,
- Figur 2: den Strömungskanal mit der Elektrodenanordnung in einem Querschnitt,
- Figur 3: den Strömungskanal mit einer die Elektrodenanordnung passierenden Gasblase,
- Figur 4: den zeitlichen Verlauf der elektrischen Kapazität der Elektrodenanordnung bei Passage einer Gasblase in qualitativer Darstellung,
- Figur 5: den zeitlichen Verlauf des Ohm'schen Widerstands bei Passage der Gasblase in qualitativer Darstellung,
- Figur 6: den zeitlichen Verlauf der Kapazität bei Passage mehrerer Gasblasen in quantitativer Darstellung,
- Figur 7: den zeitlichen Verlauf des Widerstands bei der Passage der Gasblasen in quantitativer Darstellung,
- Figur 8: die Elektrodenanordnung im Normalzustand,
- Figur 9: die Elektrodenanordnung der Figur 8 bei Auftreten einer Okklusion,
- Figur 10: eine modifizierte Elektrodenanordnung und
- Figur 11: ein Ersatzschaltbild der Elektrodenanordnung der Figur 10.

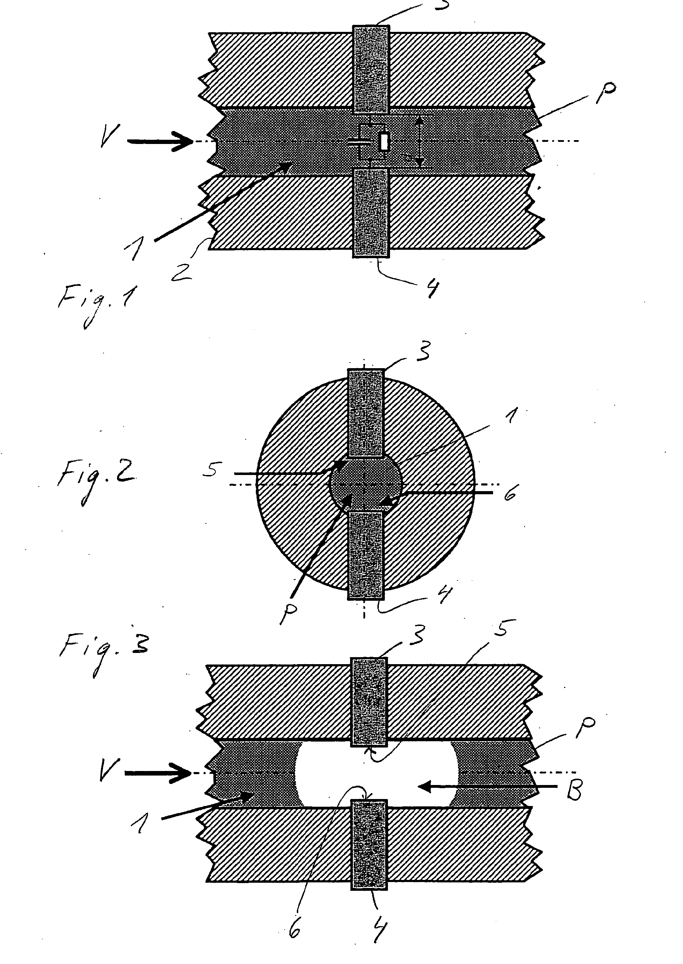

Figur 1 zeigt ein erstes Ausführungsbeispiel einer Messanordnung, die in einem Strömungskanal 1 einer Vorrichtung für die Verabreichung eines flüssigen Produkts gebildet ist, um von dem Produkt mitgeführte Gasblasen zu detektieren. Die Vorrichtung des Ausführungsbeispiels ist ein Infusionsgerät, das in der Selbstverabreichung des Produkts extern am Körper, beispielsweise in oder auf der Kleidung oder auf der Haut, tragbar ist. Derartige Infusionsgeräte sind insbesondere aus der Diabetestherapie bekannt. Insulin stellt entsprechend auch ein prominentes Beispiel für das flüssige Produkt P dar.

Der Strömungskanal 1 ist zylindrisch, im Ausführungsbeispiel kreiszylindrisch. Er wird von einer Seitenberandung 2 über seinen gesamten Umfang berandet. Die Seitenberandung 2 ist rohr- oder schlauchförmig, im Ausführungsbeispiel wird sie von einer flexiblen Kanüle gebildet. Das Produkt P wird von einer Fördereinrichtung der Verabreichungsvorrichtung aus einem Produktreservoir, beispielsweise einer Ampulle, gefördert und durchströmt den Strömungskanal 1 in Strömungsrichtung V.

In der Seitenberandung 2 sind eine erste Messelektrode 3 und eine zweite Messelektrode 4 eingesetzt und fest mit der Seitenberandung 2 verbunden. Die Messelektroden 3 und 4 überlappen einander in Strömungsrichtung V. Zwischen den Messelektroden 3 und 4 verbleibt ein rechtwinklig zu der Strömungsgeschwindigkeit V gemessener lichter Abstand d, der wenigstens 0.3mm und höchstens 3mm, vorzugsweise wenigstens 0.5 mm und höchstens 2 mm betragen sollte. Die Messelektroden 3 und 4 sind identisch gebildet, sowohl der Form als auch dem Material nach. Der Abstand d ist zwischen den einander zugewandten planparallelen Stirnflächen 5 und 6 der Messelektroden 3 und 4 gemessen. Die Messelektroden 3 und 4 ragen je geringfügig in den Strömungskanal 1 hinein, d. h. sie ragen geringfügig über die ansonsten glatte Mantelinnenfläche der Seitenberandung 2 vor, so dass die Stirnflächen 5 und 6 auf zwar flachen, aber erhabenen Plateaus gebildet sind. Die Messelektroden 3 und 4 sind Metallelektroden und sind daher mit dem Produkt P jeweils galvanisch gekoppelt. Die Elektroden sollten inert sein. Bevorzugt werden Gold- oder Platin-Elektroden verwendet.

Die aus den Messelektroden 3 und 4 bestehende Elektrodenanordnung bildet mit dem Produkt P einen elektrischen Kondensator C und einen Ohm'schen Widerstand R. Das Ersatzschaltbild der aus den Elektroden 3 und 4 und dem Produkt P gebildeten Messanordnung ist in Figur 1 ebenfalls dargestellt; es handelt sich um die Parallelschaltung des Widerstands R und der Kapazität C.

Figur 2 zeigt den Strömungskanal 1 mit der Elektrodenanordnung 3, 4 in einem Querschnitt. Die Elektrodenanordnung 3, 4 mit dem durchströmenden Fluid entspricht einem Plattenkondensator. Der Ohm'sche Widerstand R und die elektrische Kapazität C ergeben sich in bekannter Weise aus den folgenden Größen:
- dem Abstand d der Messelektroden 3 und 4,
- der Größe der identischen Stirnflächen 5 und 6,
- der relativen Dieelektrizitätszahl εᵣ des die Elektrodenanordnung 3, 4 passierenden Fluids, beispielsweise des Produkts P, und
- der spezifischen elektrischen Leitfähigkeit σ des Fluids, beispielsweise des Produkts P.

Die Förderüberwachung erfolgt durch Messung der komplexen Impedanz Z, d. h. der Kapazität C und des Widerstand R, bei Betrieb der Messelektroden 3 und 4 mit einer Wechselspannung. Die Wechselspannung sollte eine Frequenz von wenigstens 10 Hz und höchstens 200 kHz haben, was nicht nur für den Beispielfall gilt, in dem das Produkt P Insulin ist, sondern ganz allgemein für jedes Produkt P auf der Basis von Wasser als Trägerflüssigkeit. Bei Messung nur der Kapazität sollte die Frequenz wenigstens 100 Hz, bevorzugterweise wenigstens 1 kHz, betragen.

Die Messung wird mittels einer Verarbeitungseinrichtung durchgeführt, die als feste Messschaltung oder auch als programmierbare Prozessoreinrichtung gebildet sein kann und vorzugsweise Bestandteil einer Steuerungseinrichtung oder Regelungseinrichtung zur Steuerung oder Regelung der Fördereinrichtung ist.

Die Figuren 1 und 2 zeigen die Elektrodenanordnung 3, 4 im Normalzustand, in dem das Volumen zwischen den Messelektroden 3 und 4 vollständig mit flüssigem Produkt P gefüllt ist. Die relative Dieelektrizitätszahl ε_{r,P} ist groß, da der Hauptbestandteil des Produkts Wasser ist, wodurch sich trotz im Allgemeinen geringer Abmessungen der Messelektroden 3 und 4 eine einfach messbare Kapazität C = C₁ ergibt. Für einen großen Messeffekt sollte der Abstand d möglichst gering gewählt werden, beispielsweise zu d = 0.5 mm. Aufgrund der in beispielsweise Insulin enthaltenen Ionen besitzt ein derartiges Produkt P zusätzlich eine vergleichsweise gute elektrische Leitfähigkeit σ_{P}, aus der ein einfach messbarer elektrischer Widerstand R = R₁ parallel zur Kapazität C₁ resultiert. Insgesamt stellt das flüssige Produkt P daher ein stark verlustbehaftetes Dieelektrikum dar, und es ergibt sich das in Figur 1 gezeigte Parallel-Ersatzschaltbild.

Werden die Messelektroden 3 und 4 von einer im Produktstrom mitgeführten Gasblase passiert, ändern sich die Verhältnisse nahezu schlagartig.

Figur 3 zeigt die Messanordnung während der Passage einer Gasblase B, die im praktischen Betrieb eine Luftblase sein wird. Die Verhältnisse sind in der Praxis im Allgemeinen so, dass die mitgeführten Gasblasen B den Querschnitt des Strömungskanals 1 vollständig oder zumindest im Wesentlichen ausfüllen. Zumindest ist der Abstand d so bemessen, dass die Gasblasen B bei der Passage der Messelektroden 3 und 4 den Raum zwischen den Elektroden 3 und 4 ausfüllen, d. h. den Abstand d überbrücken. Die Kapazität C fällt aufgrund der für Gase, insbesondere Luft, geringen relativen Dieelektrizitätszahl ε_{r,B} ≈ 1 auf einen sehr kleinen, praktisch kaum messbaren Wert ab, d. h. C → 0. Da nicht ionisierte Gase, insbesondere Luft, nahezu ideale Isolatoren sind mit σ_{B} → 0, gilt für den Ohm'schen Widerstand R → ∞. Da in der Regel an der Mantelinnenfläche der Seitenberandung 2 sowie den Messelektroden 3 und 4 ein Flüssigkeitsfilm verbleibt, ergeben sich im praktischen Einsatz nicht diese Grenzwerte, sondern C = C₂ << C₁ und R = R₂ >> R₁.

In den Figuren 4 und 5 sind die Kapazität C und der Widerstand R beim Transit einer Luftblase B als Funktionen der Zeit t qualitativ dargestellt. Die Gasblase B füllt ab dem Zeitpunkt t₁ das Volumen zwischen den Messelektroden 3 und 4 aus und verlässt den Messbereich zum Zeitpunkt t₂. Für die Detektion von Gasblasen B sind einfache Schwellwerte für R und C ausreichend. Derartige Schwellwerte sind in die qualitativen Darstellungen der Figuren 4 und 5 eingetragen. Unterschreitet die Kapazität C den Schwellwert C_{B}, der deutlich unter C₁ und ausreichend über C₂ liegt, so stellt die Verarbeitungseinrichtung fest, dass eine Gasblase B in den Messbereich eingetreten ist. Überschreitet nach Feststellung dieses Ereignisses die Kapazität C den weiteren Schwellwert C_{P}, der deutlich über dem Wert C₂ und ausreichend unter dem Wert C₁ liegt, so stellt die Verarbeitungseinrichtung fest, dass die Gasblase B den Messbereich wieder verlassen hat. Analog sind die Verhältnisse hinsichtlich des Ohm'schen Widerstands R, dessen zeitlicher Verlauf für die Passage der gleichen Gasblase B in Figur 5 dargestellt ist.

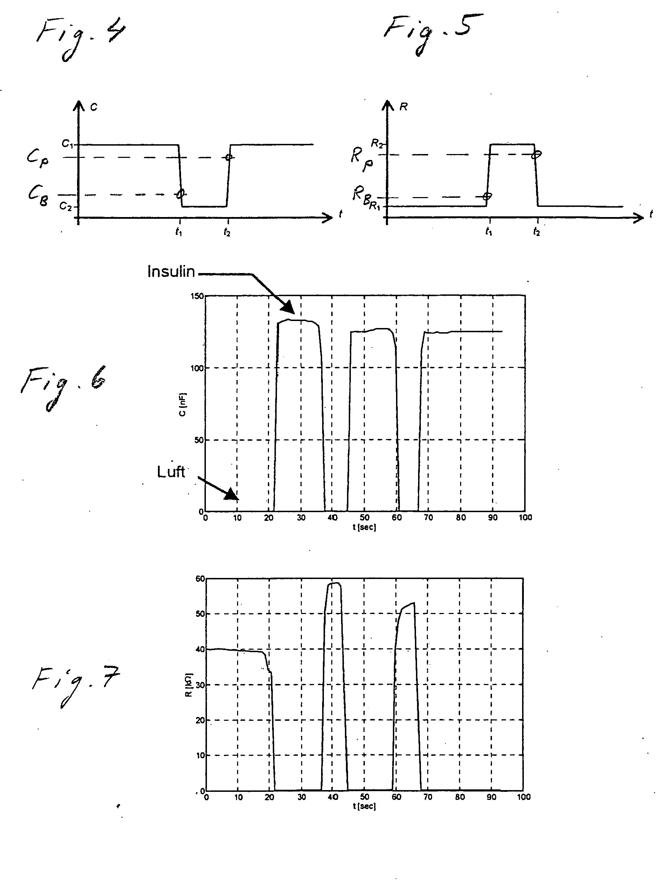

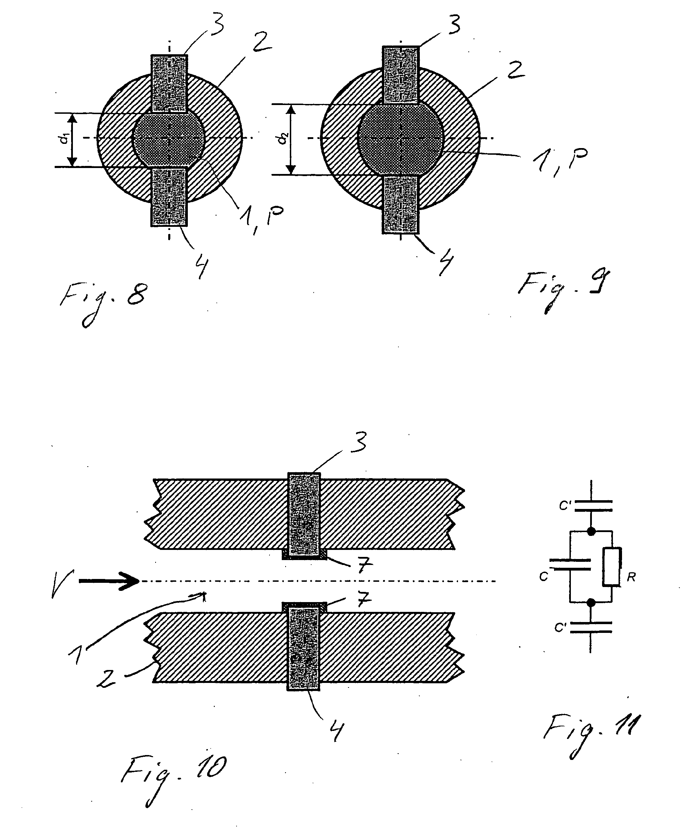

Die Figuren 6 und 7 zeigen gemessene, d. h. quantitative Verläufe der Kapazität C und des Widerstands R über der Zeit für mehrere aufeinander folgende Passagen von Gasblasen B. Gemessen wurde mit Insulin als dem flüssigen Produkt P. Bei den Gasblasen B handelt es sich um Luftblasen.

Wird die Ausdehnung der Messelektroden 3 und 4 in Strömungsrichtung V klein gewählt gegenüber den ebenfalls in Strömungsrichtung V gemessenen Längen der in der Praxis auftretenden Gasblasen B, lässt sich aus der Strömungsgeschwindigkeit v, die in der Regel konstruktiv festgelegt und somit bekannt ist, sowie der Zeitdauer Δt = t₂ - t₁ auch das Volumen der jeweiligen Gasblase B einfach errechnen zu Vol_{B} = AvΔt, wobei A die im Messbereich konstante Querschnittsfläche des Strömungskanals 1 ist.

In einer Weiterentwicklung, die in den Figuren 8 und 9 dargestellt ist, bilden die Messelektroden 3 und 4 mit der Seitenberandung 2 einen Drucksensor für die Detektion einer Okklusion oder eines Lecks. Die Seitenberandung 2 ist hierfür aus einem elastischen Material gebildet, dessen E-Modul so klein ist, dass sich die Seitenberandung 2 unter den im Falle einer Okklusion oder eines Lecks in der Praxis im Strömungskanal 1 zu erwartenden Drücken elastisch aufweitet oder entspannt. Da die Messelektroden 3 und 4 sich in Bezug auf eine zentrale Längsachse des Strömungskanals 1 diametral gegenüber liegen, wirkt sich die elastische Aufweitung oder das elastische Zusammenziehen der Seitenberandung 2 auf den Abstand d maximal aus. In Figur 8 herrscht Normaldruck, d. h. das Produkt P fließt ordnungsgemäß durch den Strömungskanal 1, der Abstand d = d₁. Figur 9 zeigt den Zustand bei Auftreten einer Okklusion stromabwärts von den Messelektroden 3 und 4. Aufgrund des erhöhten Drucks im Strömungskanal 1 weitet sich dessen elastische Seitenberandung 2 radial auf, und der Abstand d vergrößert sich auf d₂. Aufgrund der Abstandsvergrößerung von d₁ auf d₂ nimmt die Kapazität C der Elektrodenanordnung 3, 4 ab, etwa proportional zum Kehrwert der Abstandsänderung, während der Ohm'sche Widerstand R ansteigt, etwa proportional zur Abstandsänderung. Je nach der Fördertätigkeit der Fördereinrichtung, beispielsweise bei Förderung einer basalen Förderrate oder bei Förderung eines Sonderbolus, erfolgen die Abstandsänderung d₂ - d₁ und in der Folge die Änderung des Widerstands R und der Kapazität C im einen Fall langsam und quasi stetig und im anderen Fall nahezu schlagartig.

Anstatt oder zusätzlich zur Funktion eines Okklusions- oder Leckdetektors kann die Messanordnung auch nur zur Überwachung der Förderung eingesetzt werden, da sich aufgrund des Strömungswiderstandes der das Produkt führenden Querschnitte stromabwärts des Messbereichs bei der Förderung stets ein gewisser Überdruck einstellt. Gegebenenfalls kann dieser Überdruck durch besondere Maßnahmen, z. B. den Einbau eines starren Leitungsabschnitts stromabwärts des Messbereichs, gezielt erhöht werden.

Bei der Elektrodenanordnung 3, 4 der Figuren 1, 2, 3, 8 und 9 besteht zwischen den Messelektroden 3 und 4 und dem Produkt P eine direkte galvanische Kopplung, was zu einer unerwünschten Elektrolyse führen kann.

Figur 10 zeigt eine alternative Ausführung, in der die Messelektroden 3 und 4 galvanisch voneinander getrennt sind. Für die galvanische Trennung sind die Messelektroden 3 und 4 an ihren in den Strömungskanal 1 ragenden Oberflächen, d. h. im Wesentlichen ihre Stirnflächen 5 und 6, mit einer nichtleitenden Isolierung 7 versehen, die als Beschichtung gebildet ist. Nur die beiden Isolierungen 7 sind mit dem Produkt in Kontakt. Die Isolierungen 7 können beispielsweise durch Bedampfen oder Bedrucken des elektrisch gut leitenden Elektrodenmaterials mit einem Isolationsmaterial oder durch Auflaminieren einer isolierenden Folie gebildet werden. Da die Isolierung 7, wie auch immer gebildet, für jede der Messelektroden 3 und 4 eine zusätzliche, serielle Kapazität mit sich bringt und diese zusätzlichen Kapazitäten die elektrische Kapazität der Messanordnung insgesamt verringern, sollten die Isolierungen 7 möglichst dünn und ihre relative Dielektrizitätszahl möglichst groß sein.

Figur 11 zeigt das elektrische Ersatzschaltbild für die Anordnung mit den je mit einer Isolierung 7 versehenen Elektroden 3 und 4. Die durch die Isolierungen 7 zusätzlich erhaltenen Kapazitäten sind mit C' bezeichnet.

Für ein möglichst großes Messsignal ist für die Messelektroden 3 und 4 eine große Elektrodenfläche vorteilhaft. Dagegen ist zur Erfassung auch kleinerer Gasblasen B oder zur Bestimmung der Blasenvolumina eine geringe Ausdehnung der Messelektroden 3 und 4 in Strömungsrichtung V von Vorteil. Im Ergebnis sollte die Ausdehnung der Messelektroden 3 und 4 quer zu der Strömungsrichtung V, d. h. die Breite der Messelektroden 3 und 4, möglichst groß und die Ausdehnung in Strömungsrichtung V möglichst klein sein. Für die Messelektroden 3 und 4 der Ausführungsbeispiele kann die Elektrodenfläche bei in Strömungsrichtung V möglichst schmalen Messelektroden 3 und 4 dadurch vergrößert werden, dass die Messelektroden 3 und 4 sich je über einen möglichst großen Teil der Mantelinnenfläche der Seitenberandung 2 erstrecken, beispielsweise nahezu halbkreisförmig. Andererseits muss zwischen den Elektroden 3 und 4 überall ein Abstand verbleiben.

Da die Detektion von Gasblasen B lediglich eine binäre Entscheidung erfordert, während für die Detektion einer Okklusion oder eines Lecks eine quantitative Messung der Kapazität C oder des Ohm'schen Widerstands R vorteilhaft ist, können für diese unterschiedlichen Anforderungen auch getrennte Elektrodenpaare vorgesehen sein. Dabei besitzt das Elektrodenpaar 3 und 4 für die Detektion von Gasblasen B insbesondere in Strömungsrichtung V eine möglichst geringe Ausdehnung, während das Elektrodenpaar für die Okklusions- oder Leckdetektion oder nur für die Druckmessung eine möglichst große Fläche aufweist. Möglich ist ferner auch, eine Elektrode beider Elektrodenpaare zusammenzufassen, so dass sich insgesamt eine Dreielektrodenanordnung ergibt.

Insbesondere im Hinblick auf die Funktion als Blasendetektor sollte die Elektrodenanordnung 3, 4 so gestaltet sein, dass sich bei reiner Produktströmung durch den Messbereich eine elektrische Kapazität C einstellt, die wenigstens 5-mal so groß ist wie bei Passage einer den Raum zwischen den Messelektroden 3 und 4 ausfüllenden Gasblase B.

Der Ohm'sche Widerstand R sollte bei Passage einer derartigen Gasblase B wenigstens 10-mal so groß sein wie bei einer blasenfreien Produktströmung.

## Patentansprüche

1. Elektrodenanordnung mit einer ersten Messelektrode (3) und einer zweiten Messelektrode (4), die in einem Strömungskanal (1) in einem Abstand (d) voneinander angeordnet sind und mit einem flüssigen Produkt (P) bei Durchströmung des Strömungskanals (1) einen elektrischen Kondensator (C) oder Ohm'schen Widerstand (R) bilden, wobei die Elektrodenanordnung (3, 4) für die Verwendung als Sensor für die Detektion einer von dem Produkt (P) mitgeführten Gasblase (B), einer Okklusion oder eines Lecks in einer Vorrichtung für die Verabreichung des Produkts (P) vorgesehen ist.

2. Elektrodenanordnung nach Anspruch 1, **dadurch gekennzeichnet, dass** die Messelektroden (3, 4) einander in Strömungsrichtung (V) des Produkts (P) zumindest teilweise überlappen.

3. Elektrodenanordnung nach einem der vorhergehenden Ansprüche, **dadurch gekennzeichnet, dass** die erste Messelektrode (3) in einer Seitenberandung (2) des Strömungskanals (1) angeordnet ist.

4. Elektrodenanordnung nach einem der vorhergehenden Ansprüche, **dadurch gekennzeichnet, dass** die zweite Messelektrode (4) der ersten Messelektrode (3) quer zu der Strömungsrichtung (V) des Produkts (P) gegenüberliegend angeordnet ist, vorzugsweise in einer Seitenberandung des Strömungskanals (1).

5. Elektrodenanordnung nach einem der vorhergehenden Ansprüche, **dadurch gekennzeichnet, dass** die Messelektroden (3, 4) über das Produkt (P) galvanisch koppelbar sind.

6. Elektrodenanordnung nach einem der Ansprüche 1 bis 4, **dadurch gekennzeichnet, dass** wenigstens eine der Messelektroden (3, 4) mittels einer elektrischen Isolierung (7) galvanisch von dem Produkt (P) entkoppelt ist.

7. Elektrodenanordnung nach dem vorhergehenden Anspruch, **dadurch gekennzeichnet, dass** die wenigstens eine der Messelektroden (3, 4) zum Erhalt der Isolierung (7) mit einem Isoliermaterial beschichtet ist.

8. Elektrodenanordnung nach einem der vorhergehenden Ansprüche, ferner umfassend einen Spannungserzeuger zur Erzeugung einer elektrischen Potenzialdifferenz zwischen den Messelektroden (3, 4).

9. Elektrodenanordnung nach dem vorhergehenden Anspruch, **dadurch gekennzeichnet, dass** der Spannungserzeuger die Messelektroden (3, 4) mit einer Wechselspannung beaufschlagt, von vorzugsweise wenigstens 10 Hz und vorzugsweise höchstens 200 kHz.

10. Elektrodenanordnung nach einem der zwei vorhergehenden Ansprüche, **dadurch gekennzeichnet, dass** der Spannungserzeuger eine Spannungsquelle und einen Oszillator oder Generator umfasst und dass die Spannungsquelle für einen Betrieb der Messelektroden (3, 4) mit Wechselspannung über den Oszillator oder Generator mit den Messelektroden (3, 4) verbunden ist.

11. Elektrodenanordnung nach einem der vorhergehenden Ansprüche, **dadurch gekennzeichnet, dass** die Messelektroden (3, 4) nur bei Durchströmung des Strömungskanals (1) mit einer elektrischen Spannung beaufschlagt werden.

12. Elektrodenanordnung nach einem der vorhergehenden Ansprüche, **dadurch gekennzeichnet, dass** die Messelektroden (3, 4) periodisch mit einer elektrischen Spannung beaufschlagt werden.

13. Elektrodenanordnung nach einem der vorhergehenden Ansprüche, **dadurch gekennzeichnet, dass** wenigstens eine der Messelektroden (3, 4) in einer Seitenberandung (2) des Strömungskanals (1) angeordnet ist und dass die Seitenberandung (2) oder die wenigstens eine der Messelektroden (3, 4) elastisch nachgiebig ist oder sind, so dass sich der Abstand (d) zwischen den Messelektroden (3, 4) in Abhängigkeit von dem statischen Druck des Produkts (P) ändert.

14. Elektrodenanordnung nach einem der vorhergehenden Ansprüche, ferner umfassend eine weitere, dritte Messelektrode, die mit der ersten Messelektrode (3) oder der zweiten Messelektrode (4) oder noch einer weiteren, vierten Messelektrode und ferner mit dem flüssigen Produkt (P) bei Durchströmung des Strömungskanals (1) einen weiteren elektrischen Kondensator oder einen weiteren Ohm'schen Widerstand bildet, wobei die erste Messelektrode (3) und die zweite Messelektrode (4) einen Sensor für die Detektion von Gasblasen und die dritte Messelektrode mit der ihr zugeordneten Messelektrode einen Sensor für die Detektion einer Okklusion oder eines Lecks bilden.

15. Elektrodenanordnung nach dem vorhergehenden Anspruch, **dadurch gekennzeichnet, dass** eine in Strömungsrichtung (V) gemessene Überlappungslänge der ersten Messelektrode (3) und der zweiten Messelektrode (4) kleiner ist als eine in Strömungsrichtung (V) gemessene Überlappungslänge der dritten Messelektrode und der ihr zugeordneten Messelektrode.

16. Elektrodenanordnung nach einem der vorhergehenden Ansprüche, **dadurch gekennzeichnet, dass** die Messelektroden (3, 4) für die Detektion einer in einer Insulinlösung oder -suspension (P) mitgeführten Gasblase (B) in Strömungsrichtung höchstens so lang sind wie ein Volumen, dass im Bereich der Messelektroden (3, 4) den Strömungskanal (1) füllt und höchstens eine Insulineinheit (1 IU), vorzugsweise höchstens eine halbe Insulineinheit, enthält, wobei das Volumen vorzugsweise ferner wenigstens ein Zehntel einer Insulineinheit (0.1 IU) enthält.

17. Vorrichtung zur Verabreichung eines flüssigen Produkts, umfassend:
a) eine Fördereinrichtung,
b) einen Strömungskanal (1), durch den das Produkt (P) mittels der Fördereinrichtung förderbar ist,
c) eine Elektrodenanordnung mit einer ersten Messelektrode (3) und einer zweiten Messelektrode (4), die in einem Abstand (d) voneinander angeordnet und bei Durchströmung des Strömungskanals (1) über das Produkt (P) elektrisch miteinander gekoppelt sind,
d) einen Spannungserzeuger zur Erzeugung einer elektrischen Potenzialdifferenz zwischen den Messelektroden (3, 4)
e) und eine mit den Messelektroden (3, 4) verbundene Verarbeitungseinrichtung, mittels der wenigstens eines aus elektrischer Kapazität (C) und Ohm'schem Widerstand (R) der Elektrodenanordnung (3, 4) bestimmbar ist.

18. Vorrichtung nach dem vorhergehenden Anspruch, ferner umfassend einen Regler für die Fördereinrichtung, der einen mit der Verarbeitungseinrichtung verbundenen Eingang aufweist und eine Stellgröße für die Fördereinrichtung in Abhängigkeit von einer von der Verarbeitungseinrichtung erhaltenen Eingangsgröße bildet.

19. Vorrichtung nach dem vorhergehenden Anspruch, **dadurch gekennzeichnet, dass** die Verarbeitungseinrichtung dem Regler in Abhängigkeit von wenigstens einem aus elektrischer Kapazität (C) und Ohm'schem Widerstand (R) eine Störgröße aufschaltet.

20. Vorrichtung nach dem vorhergehenden Anspruch, **dadurch gekennzeichnet, dass** die Verarbeitungseinrichtung bei Detektion einer Gasblase (B) die Störgröße als vorgegebenen Wert bildet.

21. Vorrichtung nach einem der vorhergehenden Ansprüche, **dadurch gekennzeichnet, dass** die Verarbeitungseinrichtung ein Addierwerk für die Bestimmung der Anzahl von nacheinander detektierten Gasblasen (B) umfasst.

22. Vorrichtung nach dem vorhergehenden Anspruch in Kombination mit Anspruch 17, **dadurch gekennzeichnet, dass** mittels der Verarbeitungseinrichtung die Anzahl der Gasblasen (B) für ein vorgegebenes Zeitintervall oder einen abgrenzbaren Verabreichungsvorgang bestimmbar ist und diese Anzahl dem Regler als die Eingangsgröße übermittelt wird.

23. Vorrichtung nach einem der zwei vorhergehenden Ansprüche in Kombination mit Anspruch 17, **dadurch gekennzeichnet, dass** die Verarbeitungseinrichtung die Anzahl der Gasblasen (B) mit einer vorgegebenen Vergleichszahl vergleicht und die Anzahl der Gasblasen (B) dem Regler erst dann als die Eingangsgröße übermittelt, wenn die ermittelte Anzahl wenigstens so groß ist wie die Vergleichszahl.

24. Vorrichtung nach einem der vorhergehenden Ansprüche, **dadurch gekennzeichnet, dass** die Verarbeitungseinrichtung ein Rechenwerk für die Bestimmung des Volumens einer die Messelektroden (3, 4) passierenden Gasblase (B) umfasst.

25. Vorrichtung nach dem vorhergehenden Anspruch in Kombination mit Anspruch 18, **dadurch gekennzeichnet, dass** die Verarbeitungseinrichtung dem Regler das Volumen der Gasblase (B) als die Eingangsgröße übermittelt.

26. Vorrichtung nach einem der zwei vorhergehenden Ansprüche, **dadurch gekennzeichnet, dass** mittels der Verarbeitungseinrichtung ein Zeitintervall (t₁ - t₂) zwischen zwei zeitlich aufeinander folgenden Änderungen von wenigstens einem aus elektrischer Kapazität (C) und Ohm'schem Widerstand (R) der Elektrodenanordnung (3, 4) bestimmbar ist.

27. Vorrichtung nach dem vorhergehenden Anspruch, **dadurch gekennzeichnet, dass** die Verarbeitungseinrichtung einen Datenspeicher für die Speicherung eines Kennwerts (C_{P}, C_{B}, R_{P}, R_{B}) für das wenigstens eine aus elektrischer Kapazität (C) und Ohm'schem Widerstand (R) aufweist und dass die Verarbeitungseinrichtung zur Bestimmung eines Anfangszeitpunkts (t₁) und eines Endzeitpunkts (t₂) einer Passage einer Gasblase (B) einen Messwert (C₁, C₂, R₁, R₂) für das wenigstens eine aus elektrischer Kapazität (C) und Ohm'schem Widerstand (R) mit dem Kennwert (C_{P}, C_{B}, R_{P}, R_{B}) vergleicht.

28. Vorrichtung nach einem der zwei vorhergehenden Ansprüche, **dadurch gekennzeichnet, dass** mittels des Rechenwerks aus dem Zeitintervall (t₁ - t₂), einer vorgegebenen oder ermittelten Strömungsgeschwindigkeit des Produkts (P) und einer vorgegebenen oder ermittelten Querschnittsfläche, die der Strömungskanal (1) im Bereich der Elektrodenanordnung (3, 4) aufweist, das Volumen einer die Elektrodenanordnung passierenden Gasblase (B) ermittelbar ist.

29. Vorrichtung nach einem der fünf vorhergehenden Ansprüche, **dadurch gekennzeichnet, dass** das Rechenwerk ein Addierwerk für die Addition der Volumina von mehreren nacheinander detektierten Gasblasen (B) umfasst und die Verarbeitungseinrichtung dem Regler das Gesamtvolumen dieser Gasblasen (B) als die Eingangsgröße übermittelt.

30. Vorrichtung nach dem vorhergehenden Anspruch, **dadurch gekennzeichnet, dass** die Verarbeitungseinrichtung das Gesamtvolumen für ein vorgegebenes Zeitintervall oder einen abgrenzbaren Verabreichungsvorgang bestimmt und dieses Gesamtvolumen dem Regler als die Eingangsgröße übermittelt.

31. Vorrichtung nach einem der zwei vorhergehenden Ansprüche, **dadurch gekennzeichnet, dass** die Verarbeitungseinrichtung das ermittelte Gesamtvolumen mit einem vorgegebenen Vergleichsvolumen vergleicht und das ermittelte Gesamtvolumen dem Regler erst dann als die Eingangsgröße übermittelt, wenn es wenigstens so groß ist wie das Vergleichsvolumen.

32. Vorrichtung nach einem der vorhergehenden Ansprüche, **dadurch gekennzeichnet, dass** die Verarbeitungseinrichtung ein Zeitintervall ermittelt oder in einem Datenspeicher der Verarbeitungseinrichtung ein vorgegebener, das Zeitintervall repräsentierender Wert gespeichert ist, wobei das Zeitintervall dasjenige Zeitintervall ist, das eine detektierte Gasblase (B) benötigt, bis sie vom Zeitpunkt der Detektion gerechnet ein stromabwärtiges Ende des Strömungskanals (1), an dem der Strömungskanal (1) mündet, erreicht.

33. Vorrichtung nach dem vorhergehenden Anspruch in Kombination mit Anspruch 18, **dadurch gekennzeichnet, dass** dem Regler das Zeitintervall oder eine daraus abgeleitete Größe aufgebbar ist und die Fördereinrichtung mittels des Reglers in Abhängigkeit von dem Zeitintervall steuer- oder regelbar ist.

34. Vorrichtung nach einem der vorhergehenden Ansprüche, ferner umfassend eine mit der Verarbeitungseinrichtung verbundene und in Abhängigkeit von einem Ausgangssignal der Verarbeitungseinrichtung aktivierbare Alarmeinrichtung zur Ausgabe eines akustischen, optischen oder taktilen Alarmsignals.

35. Vorrichtung nach einem der vorhergehenden Ansprüche, **dadurch gekennzeichnet, dass** die Elektrodenanordnung (3, 4) eine Elektrodenanordnung nach einem der Ansprüche 1 bis 15 ist.
